# EUROPEAN PATENT SPECIFICATION

(11) **EP 0 370 870 B1**
(45) Date of publication and mention of the grant of the patent: **24.05.1995**
(21) Application number: 89403153.3
(22) Date of filing: 17.11.1989
(51) Int. Cl.: G01N 33/00

(54) **Process for producing low-concentration gas mixtures, and apparatus for producing the same**
Verfahren zum Herstellen von Gasgemischen geringer Konzentration und Vorrichtung zu ihrer Erzeugung
Procédé pour préparer des mélanges de gaz ayant une concentration faible et appareil pour leur production

(30) Priority: 21.11.1988 EP 88402920
(43) Date of publication of application: 30.05.1990
(73) Proprietor: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventor: Mettes, Jacques, Tsucuba Gun Ibaraki-Pref. 300-26 (JP); Kimura, Takako, Tsucuba Gun Ibaraki-Pref. 300-26 (JP); Schack, Michael, c/o AIR LIQUIDE GMBH, D-4000 Düsseldorf 1 (DE)
(74) Representative: Vesin, Jacques

(56) References cited:
- DE-A- 1 548 880
- DE-A- 2 904 872
- FR-A- 2 321 122
- GB-A- 2 040 715
- US-A- 3 533 272
- PATENT ABSTRACTS OF JAPAN, vol. 9, no. 314 (P-412)[2037], 10 December 1985#

## Description

The present invention relates to the manufacture of low-concentration gas mixtures and, more particularly, to a process and an apparatus for producing gas mixtures by diluting a material gas in one or more stages, for testing, calibrating, or the like, highly sensitive analytical instruments, such as those used for sub-ppb analysis with Atmospheric Pressure Ionization Mass Spectrometry (APIMS).

A variety of gases are used in manufacturing semiconductor devices such as LSIs. These gases contain impurities. These impurities have been suspected to have an adverse influence on the characteristics of the LSIs. Hence, it is demanded that the gases be as pure as possible. This demand grows stronger, along with the increase in the integration density of LSIs. To meet this demand, a high-accuracy and reliable analysis of gases is required.

The techniques commonly used for analyzing such gases for determining the impurity contents thereof are : gas chromatography (GC), gas chromatography-mass spectroscopy (GC-MS), and Fourier transformed infrared spectroscopy (FTIR). The detection limit of these techniques are, however, 1 to 10 ppb at best. In view of this, these analytical techniques cannot be said to determine the impurity content of the gases as sensitively as is required in the manufacture of LSIs.

Furthermore, in order to conduct a successful quantitative analysis on a particular species in a sample gas by a non-absolute method such as mass spectroscopic method, it is necessary to make a calibration curve by using standard gas mixtures containing the species to be analysed. Theses standard mixtures can be prepared by diluting a gas mixture of a known high concentration of the impurity, with a diluent gas. The concentration of the species in the final mixture should be preferably in the same range as that in the sample gas to be analyzed. When the concentration of the sample gas is very low, the following factors determine the accuracy of the analysis :
a. Detection limit of the analytical instrument
b. Purity of the diluent gas
c. Mixing techniques

When the detection limit of the analytical instrument is in lower orders, it is difficult to obtain factors b and c, both in the comparable orders.

Recently, a highly sensitive analytical instrument, which is called atmospheric-pressure ionization mass spectrometer (APIMS) has been developed. This instrument can determine the contents of molecular species down to 10 ppt. Therefore, it has become desirable to produce standard gas mixtures in the low concentration range.

Low-concentration standard gas mixtures, which is used as a calibration gas in analyzing extremely pure gases, could be produced by diluting a high-concentration standard gas in one or a plurality of stages. In order to produce a low-concentration standard gas, continuously at a desired flow rate and pressure, the two-stage dilution method, for example, is employed. In this method, first the high-concentration standard gas is diluted with a diluent gas of the same kind of the sample gas to a predetermined medium lower concentration, then most of this medium concentration mixture is discarded, and the remaining fraction of said gas is further diluted with the diluent gas. The concentration of the species within this low concentration, final standard gas must be controlled accurately. For this purpose, various devices such as mass flow controllers, pressure regulator, must be used to control the flow rates of the material gas and the diluent gases. As soon as low concentrations should be made of species that make part of our natural environment, the use of regulation devices like mass flow controllers, pressure regulators, etc would give serious limitations on the lower limits that can be achieved.

It is known from Patent Abstract of Japan, 9, n^{o} 314 (P. 412) (2037), 10/12/1985, JP-A-60 143738, a two-steps dilution process of gases, wherein the pressure of a raw gas and a diluting gas are kept substantially constant, by mixing them in a buffer which is alternately fed with each gas, said mixture being again diluted with the diluting gas the same way.

In order to prepare low-concentration standard gas mixtures in dynamic mode, the inventors have cound that it is very important to maintain the diluent gas lines and the mixing lines free from contamination. The devices necessarily used to control the flow rates and pressures of the material gas and the diluent gas absorb and degas contaminants and are unavoidably sources of contaminants. The contaminants, if released from these devices, greatly change the concentration of the low-concentration standard gas mixture or add additional species in an uncontrolled manner. Consequently, no correct calibration curves can be obtained. Without correct calibration curves, even a high-accuracy analytical instrument such as an APIMS cannot analyze gases with such an accuracy as is required now in manufacturing LSIs.

One object of the present invention is to provide a process for producing low-concentration gas mixtures desirable as standard gas mixtures for analyzing high-purity gases, in which diluent gas lines and mixing lines are maintained free from contamination.

Another object of the invention is to provide an apparatus for performing the process efficiently, thereby producing such low-concentration gas mixtures.

According to the present invention, there is provided an apparatus as claimed in anyone of claims 1 to 5, as well as a process which is carried out with this apparatus, according to anyone of claims 6 to 17.

The basic concept of the present invention is to provide a process and related apparatus wherein, as soon as the high purity diluent gas is generated, no further contaminants such as particles and/or gaseous impurities are added during the further mixing and/or diluting steps of the process. That means that all the devices used for carrying out those steps are able to generate no additionnal contaminants. Those devices are generally selected among pipes, such as electropolished pipes, and restriction means, such as needle valves (controllable flowrate), calibrated orifices, small diameter pipes, with appropriate diameters as well as appropriate ratios of diameters when different flow-rates and pressures have to be handled in different lines, the selection of the appropriate ratio of diameters being well-known by the man skilled in the art.

The present invention can be applied to a multi-stage diluting process, wherein a zero gas, i.e., a high-purity diluent gas to be mixed with a high-concentration standard gas to generate a gas mixture, is divided into n flow portions, these flow portions of the zero gas being sequentially added to a mixture gas such that the nth flow of the zero gas is added to a portion of the gas mixture diluted in the (n-1)th stage.

This multi-stage diluting process comprises the steps of : controlling the pressure of a raw gas ; purifying the raw gas, thereby generating a high-purity diluent gas ; dividing the high-purity diluent gas into n flow portions ; controlling the flow rate of a high-concentration standard gas ; mixing a first flow portion of the high-purity diluent gas with the high-concentration standard gas, thereby generating a first medium-concentration gas mixture ; dividing the first medium-concentration gas mixture into a first flow and a second flow ; mixing said first flow of said first medium-concentration gas mixture with a second flow portion of the high-purity diluent gas, thereby generating a second medium concentration gas mixture ; dividing said second medium concentration gas mixture into a first flow and a second flow ; repeating the two above mentionned mixing and dividing steps to generate a first flow and a second flow of a (n-1)th medium concentration gas mixture ; mixing the first flow of the (n-1)th medium-concentration gas mixture with the nth flow portion of the high purity diluent gas, thereby generating a low concentration gas mixture ; and controlling the pressure of the second flow of each of the first to (n-1) th medium-concentration gas mixtures, the pressure of said second flow of the (m)th medium concentration gas mixture (1<m<n-1) being smaller than that of the (m-1)th medium concentration gas mixture. Preferably, if it is not recovered or recycled, the second flow of each of the medium concentration gas mixture is vented.

Still further, according to the invention there is provided an apparatus for producing low-concentration gas mixtures, comprising : means for controlling the pressure of a raw gas ; means for purifying the raw gas, thereby generating a high-purity diluent gas ; means for dividing said high-purity diluent gas into n flow portions, each portion flowing through first restricting means ; means for controlling the flow rate of a high-concentration standard gas ; means for mixing a first flow-portion of the high-purity diluent gas with the high-concentration standard gas, thereby generating a first medium-concentration gas mixture ; means for dividing the first medium-concentration gas mixture into a first flow and a second flow, said first flow passing through second restriction means ; means for mixing said first flow of the first medium-concentration gas mixture with a second flow portion of the high-purity diluent gas, thereby generating a second medium concentration gas mixture ; means for dividing said second medium-concentration gas mixture into a first flow and a second flow ; means for mixing and means for dividing to generate a first flow and a second flow of a (m)th medium concentration gas mixture with 1<m<n-1, means for mixing the first flow of the (n-1)th flow of the medium-concentration gas mixture with the nth flow portion of the high-purity diluent gas, generating a low concentration gas mixture ; means for controlling the pressure of said low concentration gas mixture; means for controlling the pressure of the second flow of each of the first to (n-1)th medium-concentration gas mixtures.

Preferably, the apparatus according to the invention will further comprise, means for venting the second flow of each of the medium-concentration gas mixtures. Alternatively, it may comprise means for recovering or recycling said second flow of each of the medium concentration gas mixtures.

No devices which might potentially be contaminant sources are used in the zero gas lines or the mixing lines in the process or apparatus according to the present invention. Orifices or needle valves, which are not contaminant sources, are generally used in the zero gas lines or mixing lines. In the conventional prior art process and apparatus, mass flow controllers and pressure regulators are placed in the zero gas lines and the mixing lines in order to control the flow rates and pressures of the low-concentration gas mixture. According to the present invention, mass flow controllers and pressure regulators are placed upstream of the gas-purifying means, in the high-concentration standard gas lines, the contamination of which is not so problematical, and in the gas-venting lines which are downstream the gas lines of the system according to the invention which are thus prevent from contamination. This specific arrangement of the mass flow controllers and the pressure regulators is based on the inventors'finding that the controllers and regulators can control correctly the flow rate and pressure of the low-concentration gas mixture.

In the body of the present specification, various steps are defined which may have the following meanings according to the invention :

Controlling the pressure of the raw gas is preferably carried out either before the purification step, by means of a pressure regulator or after the purification step, by means of a back pressure regulator.

Controlling the flow rate of a high concentration standard gas may be preferably accurately carried out either by means of a mass flow controller or a neddle valve associated to a pressure regulator.

Controlling the pressure of the low concentration gas mixture, i.e. the mixture adapted to flow in the analyzer, such as APIMS, is carried out preferably :
- with a back pressure regulator connected at the end of the pipe delivering the low concentration gas mixture, thus avoiding about any contamination of the pipe.
- with the analysis apparatus itself which is sometimes able to control itself said pressure (e.g. when the analysis is carried out at atmospheric pressure).
- with a pressure regulator connected at the output of the analysis apparatus, thus providing no contamination of the low concentration gas mixture.

In the present specification the terms high purity diluent gas or zero gas are equivalent. Such high purity gas is obtained by well-known techniques such as catalysis, chemical conversion, gettering, ambient temperature physical absorption, cryogenic absorption, filtering with molecular sieves,... or a combinaison of those methods, in order to remove about any impurities such as particles and gaseous impurities,...

Various appropriate methods are disclosed for example in the article of F.W. Giaccobbe and G.S. Khan, both of American Air Liquide Inc., entilted "Production of ultra high purity nitrogen" - Solid state Technology-July 1987 -.

It is also to be understood that a high concentration standard gas means preferably a gas containing only one species. But according to the invention, it also means a mixture of a plurality of species, whatever the relative proportions of each species are.

This invention will be more fully understood from the following detailed description when taken in conjunction with the accompanying drawings, in which :
Fig. 1 is a flow diagram showing a process of producing low-concentration gas mixtures, in which a high-concentration standard gas is diluted in two stages; and
Fig. 2 is also a flow diagram showing a process of producing a low-concentration gas mixture, in which a high-concentration standard gas is diluted in n stages.

A variety of embodiments of the present invention will now be described, with reference to the accompanying drawings.

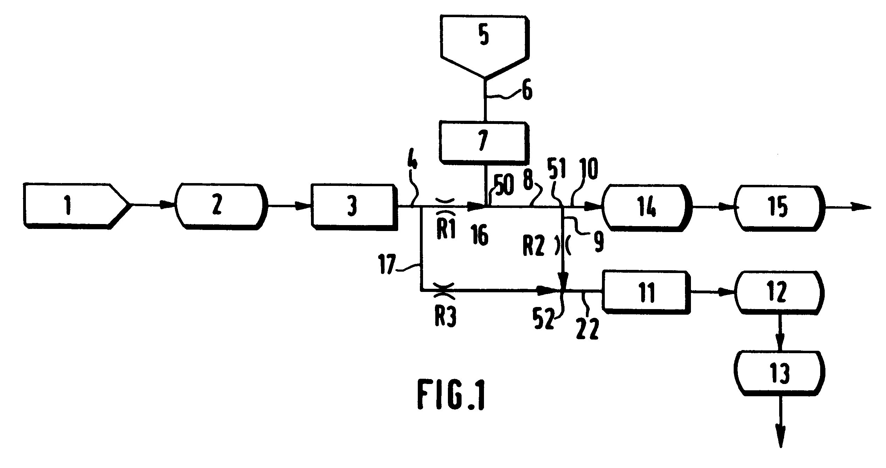

Fig. 1 is a flow diagram showing a process for producing low-concentration gas mixtures, wherein a high-concentration standard gas is diluted in two stages. As is shown in Fig. 1 the raw gas is supplied from a raw gas source 1 via a pressure regulator 2 to a purifier 3. The output gas from purifier 3, i.e., zero gas, is divided by the branching tube 4 into a first zero-gas portion 16 and a second zero-gas portion 17. The first zero-gas portion 16 passes through a restriction device R1 such as an orifice or a needle valve, and is mixed with a high-concentration standard gas 6 supplied from a standard gas source 5, through the mass flow controller 7 and the T-junction 50 thus diluting this gas mixture 6. As a result, a medium-concentration gas mixture 8 is prepared. The flow rate of the high-concentration standard gas mixture 6 is controlled by the mass flow controller 7 connected to the output of the standard gas source 5.

The medium-concentration gas mixture 8 is divided in the branching tube 51 into a first and a second streams 9 and 10. The ratio of the flow rates of the streams 9 and 10 is, for example, 1 : 100. This ratio can be reasonnably varied between about 1:20 and 1:500 (the range of the ratio is limited by the measurement accuracy of the two flow rates).

The first stream 9 of the gas mixture 8 is supplied through the restriction device R2 which is preferably either an orifice or a needle valve. Meanwhile the second zero-gas portion 17 is supplied via the restriction device R3 (either an orifice or a needle valve), and is mixed in the branching tube 52 with the first stream 9 of the gas mixture 8, thus diluting the medium concentration gas mixture to generate a low-concentration gas mixture 22. As a result, a standard gas mixture 22 having a low concentration is prepared. This standard gas mixture can be used for various purposes. For example, it is supplied to a gas analytical instrument 11.

The pressure of the low-concentration gas mixture 22 can be controlled by means of a back pressure regulator 12 connected to the outlet port of the instrument 11, or by opening the outlet port of the instrument 11 to the atmosphere. In either cases, the flowmeter 13 measures the flow rate of the low-concentration gas mixture 22 before this gas mixture is discarded out of the system.

In the meantime, the second stream 10 is discarded through the back-pressure regulator 14 and the flowmeter 15. The back-pressure regulator 14 is used to adjust the upstream pressure of the gas mixture 8.

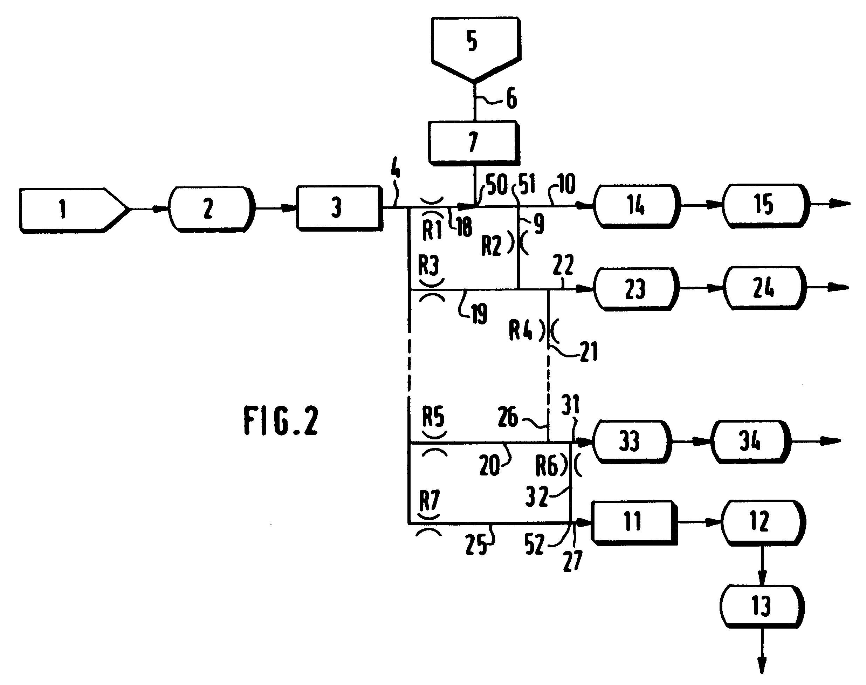

Fig. 2 is a flow diagram showing a process for producing low-concentration gas mixtures, in which a high-concentration standard gas is diluted in n stages. The same devices of fig. 1 bear the same references. As is shown on Fig. 2, the zero gas 4 is divided into a first, second,... n-1 and an zero-gas portions 18, 19,...,20 and 23. The first flow 21 of the medium-concentration gas mixture, which has been prepared in the second dilution stage, (but which is not yet a low-concentration gas mixture according to the present embodiment) is diluted with a third zero-gas portion, etc... up to obtain the first flow of the (n-2) medium-concentration gas mixture 26. The second flow 22 of the medium concentration gas mixture, which has been prepared in the second dilution stage, is discarded through the back-pressure regulator 23 and the flow-meter 24, thereby adjusting the upstream pressure with the regulator 23. The restriction devices R1 to R7, all shown on Fig. 2, are each either an orifice or a needle valve. The said first flow 26 is diluted with the (n-1) zero-gas portion 20, generating the (n-1) medium-concentration gas mixture which is divided in a first flow 32 and a second flow 31, said second flow 31 being vented through the back pressure regulator 33 and the flow meter 34. The said first flow 32 is further diluted in the branching tube 52 with the n zero gas portion 25 (or nth flow portion) generating the low-concentration gas mixture 27 introduced in the analyzer 11 as explained on Fig. 1.

The present invention is not limited to the embodiments which are shown on Figs. 1 and 2 which have been described above. As explained above, in the process for producing a low-concentration gas mixtures according to the present invention, no potential contaminant sources are used which are connected to the zero gas lines or mixing lines which are located downstream of the purifier. Hence, the process according to the invention can produce low-concentration gas mixtures which have a desired accurate concentration of one or several species in a raw gas.

## Claims

1. An apparatus for producing low-concentration gas mixtures, comprising :
- means for controlling the pressure (2) of a raw gas ;
- means for purifying the raw gas (3), thereby generating a high-purity diluent gas, said means (3) being placed downstream of means (2) ;
- means for dividing (4) said high-purity diluent gas in a first and second portion, said second portion flowing through first restricting means (R1) ;
- second restricting means (R3) through which said first portion flows ;
- means for controlling the flow rate (7) of a high-concentration standard gas ;
- means for mixing (50) the first portion of the high-purity diluent gas and the high-concentration standard gas, thereby generating a medium-concentration gas mixture ;
- means for dividing (51) the medium-concentration gas mixture into a first flow and a second flow, said first flow passing through third restricting means (R2) ;
- means for diluting (52) the medium concentration gas mixture of the first flow with said second portion of the high-purity diluent gas, thereby generating a low-concentration gas mixture ;
- means for controlling the pressure (14,15) of said second flow portion of the gas mixture, thereby controlling the pressure of said first portion ;
- means for controlling the pressure (12,13) of said low-concentration gas mixture.

2. The apparatus according to claim 1, characterized in that said flow-restricting means (R1, R3) is either a calibrated orifice or a needle valve.

3. An apparatus for producing low-concentration gas mixtures, comprising :
- means for controlling the pressure (2) of a raw gas ;
- means for purifying the raw gas (3), thereby generating a high-purity diluent gas, said means (3) being placed downstream of means (2) ;
- means for dividing (4) said high-purity diluent gas into n flow portions, each portions, each portion flowing through first restricting means (R1, R3, R5, R7) ;
- means for controlling the flow rate (7) of a high-concentration standard gas ;
- means for mixing (50) a first flow portion of the high-purity diluent gas with the high-concentration standard gas, thereby generating a first medium-concentration gas mixture ;
- means for dividing (51) the first medium-concentration gas mixture into a first flow and a second flow, said first flow passing through second restrictions means (R2) ;
- means for mixing said first flow of the first medium-concentration gas mixture with a second flow portion of the high-purity diluent gas, thereby generating a second medium-concentration gas mixture ;
- means for dividing said second medium-concentration gas mixture into a first flow and a second flow ;
- means for mixing and means for dividing to generate a first flow and a second flow of a (n-1)th medium-concentration gas mixture ;
- means for mixing (52) the first flow of the (n-1)th flow of the medium-concentration gas with the nth flow portion of the high-purity diluent gas, thereby generating a low-concentration gas mixture ;
- means for controlling the pressure (12,13) of said low-concentration gas mixture ;
- means for controlling the pressure (14,15,23,24,33,34) of the second flow of each of the first to (n-1)th medium-concentration gas mixtures.

4. The apparatus according to claim 3, wherein the first and second flow-restricting means (R1, R2, R3, R5, R7) are either calibrated orifices or needle valves.

5. The apparatus according to claim 3 or 4, wherein it further comprises means for venting the second flow of each of the medium concentration gas mixtures.

6. A process for producing low-concentration gas mixtures, using the apparatus according to one of claims 1 or 2 comprising the steps of:
- Controlling the pressure of a raw gas, then
- purifying the raw gas, thereby generating a high purity diluent gas ;
- dividing said high purity diluent gas in a first and second portions ;
- Controlling the flow rate of at least one high-concentrtion standard gas ;
- mixing said first portion of said high-purity diluent gas and the high-concentration standard gas, thereby generating a medium-concentration gas mixture ;
- dividing the medium-concentration gas mixture into a first flow and a second flow ;
- diluting the gas mixture of the first flow with said second portion of the high purity diluent gas, thereby generating a low-concentration gas mixture ;
- controlling the pressure of said second flow of the medium gas mixture ; and controlling the pressure of said low concentration gas mixture.

7. The process according to claim 6, further comprising the step of restricting the flow of the first portion of the high-purity diluent gas to be mixed with the high-concentration standard gas.

8. The process according to claim 6 or 7, further comprising the step of restricting said first flow of the medium-concentration gas mixture.

9. The process according to one of claims 6 to 8, further comprising the step of restricting the flow of said second portion of the high-purity diluent gas.

10. The process according to one of claims 7 to 9 wherein each step of restricting the flow is carried out by restriction means selected among a calibrated orifice or a needle valve.

11. The process according to one of claims 6 to 10, further comprising the step of venting said second flow of medium concentration gas mixture.

12. A process for producing low-concentration gas mixtures, using the apparatus according to one of claims 3 to 5, comprising the steps of:
- controlling the pressure of a raw gas, then
- purifying the raw gas, thereby generating a high-purity diluent gas ;
- dividing the high-purity diluent gas into n flow portions ;
- controlling the flow rate of at least one high-concentration standard gas ;
- mixing a first flow portion of the high-purity diluent gas with the high-concentration standard gas, thereby generating a first medium-concentration gas mixture ;
- dividing the first medium-concentration gas mixture into a first flow and second flow ;
- mixing said first flow of said first medium-concentration gas mixture with a second flow portion of the high-purity diluent gas, thereby generating a second medium-concentration gas mixture ;
- dividing said second medium-concentration gas mixture into a first flow and a second flow ;
- repealing (m) times the two above mentionned mixing and dividing steps to generate a first and a second flows of a (m)th medium-concentration gas mixture, with m varying from 2 to n-1 ;
- mixing the first flow of the (n-1)th flow of the (n-1)th flow of the medium-concentration gas with the nth flow portion of the high-purity diluent gas, thereby generating a low-concentration gas mixture ;
- controlling the pressure of said low-concentration gas mixture ;
- controlling the pressure of the second flow of each of the first to (n-1)th medium-concentration gas mixtures, the pressure of said second flow of the (m)th medium-concentration gas mixture (1<m<n-1) being smaller than that of the (m-1) th medium-concentration gas mixture.

13. The process according to claim 12, further comprising the step of restricting the flow of each the n flow portions of the high purity diluent gas.

14. The process according to claim 12 or 13, further comprising the step of restricting the flow of said first flow of each of said medium-concentration gas mixture.

15. The process according to one of claims 12 to 14 further comprising the step of venting the second flow of each of the medium-concentration gas mixtures.

16. The process according to claims 13 or 14, wherein each step of restricting the flow is carried out by restriction means selected among calibrated orifices or needle valves.

17. The process according to any one of claims 6 to 16, further comprising the steps of controlling the flowrate of a plurality of high concentration standard gases and mixing said high concentration standard gases to generate a high concentration standard gas mixture which respective proportions by volume are controlled, said mixture being further mixed with said first portion of said high purity diluent gas.

## Patentansprüche

1. Vorrichtung zur Erzeugung von Gasgemischen niedriger Konzentration, mit
- einer Einrichtung (2) zum Steuern des Drucks eines Rohgases;
- einer Einrichtung (3) zum Reinigen des Rohgases zur Erzeugung eines Verdünnungsgases hoher Reinheit, wobei die Einrichtung (3) stromabwärts von der Einrichtung (2) angeordnet ist;
- einer Einrichtung (4) zum Aufteilen des Verdünnungsgases hoher Reinheit in einen ersten und einen zweiten Teil, wobei der zweite Teil durch eine erste Drosseleinrichtung (R1) strömt;
- einer zweiten Drosseleinrichtung (R3), durch die der erste Teil strömt;
- einer Einrichtung (7) zum Steuern des Durchflusses eines Standardgases hoher Konzentration;
- einer Einrichtung (50) zum Mischen des ersten Teils des Verdünnungsgases hoher Reinheit und des Standardgases hoher Konzentration zur Erzeugung eines Gasgemisches mittlerer Konzentration;
- einer Einrichtung (51) zum Aufteilen des Gasgemisches mittlerer Konzentration in einen ersten Strom und einen zweiten Strom, wobei der erste Strom eine dritte Drosseleinrichtung (R2) durchsetzt;
- einer Einrichtung (52) zum Verdünnen des Gasgemisches mittlerer Konzentration des ersten Stroms mit dem zweiten Teil des Verdünnungsgases hoher Reinheit zur Erzeugung eines Gasgemisches niedriger Konzentration;
- einer Einrichtung (14, 15) zum Steuern des Drucks des zweiten Stromteils des Gasgemisches zum Steuern des Drucks des ersten Teils;
- einer Einrichtung (12, 13) zu Steuern des Drucks des Gasgemisches niedriger Konzentration.

2. Vorrichtung nach Anspruch 1, dadurch gekennzeichnet, daß die Drosseleinrichtungen (R1, R3) entweder eine kalibrierte Öffnung oder ein Nadelventil sind.

3. Vorrichtung zum Erzeugen von Gasgemischen niedriger Konzentration mit
- einer Einrichtung (2) zum Steuern des Drucks eines Rohgases;
- einer Einrichtung (3) zum Reinigen des Rohgases zur Erzeugung eines Verdünnungsgases hoher Reinheit, wobei die Einrichtung (3) stromabwärts von der Einrichtung (2) angeordnet ist;
- einer Einrichtung (4) zum Aufteilen des Verdünnungsgases hoher Reinheit in n Stromteile, wobei jeder Teil durch erste Drosseleinrichtungen (R1, R3, R5, R7) strömt;
- einer Einrichtung (7) zum Steuern des Durchflusses eines Standardgases hoher Konzentration;
- einer Einrichtung (50) zum Mischen eines ersten Stromteils des Verdünnungsgases hoher Reiheit mit dem Standardgas hoher Konzentration zur Erzeugung eines ersten Gasgemisches mittlerer Konzentration;
- einer Einrichtung (51) zum Aufteilen des ersten Gasgemisches mittlerer Konzentration in einen ersten Strom und einen zweiten Strom, wobei der erste Strom eine zweite Drosseleinrichtung (R2) durchfließt;
- einer Einrichtung zum Mischen des ersten Stroms des ersten Gasgemisches mittlerer Konzentration mit einem zweiten Stromteil des Verdünnungsgases hoher Reiheit zur Erzeugung eines zweiten Gasgemisches mittlerer Konzentration;
- einer Einrichtung zum Aufteilen des zweiten Gasgemisches mittlerer Konzentration in einen ersten Strom und einen zweiten Strom;
- einer Einrichtung zum Mischen und einer Einrichtung zum Aufteilen zur Erzeugung eines ersten Stroms und eines zweiten Stroms in einem (n-1)-ten Gasgemisch mittlerer Konzentration;
- einer Einrichtung (52) zum Mischen des ersten Stroms des (n-1)-ten Stroms des Gasgemisches mittlerer Konzentration mit dem n-ten Stromteil des Verdünnungsgases hoher Reinheit zur Erzeugung eines Gasgemisches niedriger Konzentration;
- einer Einrichtung (12, 13) zum Steuern des Drucks des Gasgemisches niedriger Konzentration;
- einer Einrichtung (14, 15, 23, 24, 33, 34) zum Steuern des Drucks des zweiten Stroms jedes der ersten bis (n-1)-ten Gasgemische mittlerer Konzentration.

4. Vorrichtung nach Anspruch 3, wobei die ersten und zweiten Drosseleinrichtungen (R1, R2, R3, R5, R7) entweder kalibrierte Öffnungen oder Nadelventile sind.

5. Vorrichtung nach Anspruch 3 oder 4, zusätzlich umfassend eine Einrichtung zum Auslassen des zweiten Stroms jedes der Gasgemische mittlerer Konzentration.

6. Verfahren zur Erzeugung von Gasgemischen niedriger Konzentration unter Verwendung der Vorrichtung nach einem der Ansprüche 1 oder 2, umfassend die Schritte:
- Steuern des Drucks eines Rohgases, daraufhin
- Reinigen des Rohgases zur Erzeugung eines Verdünnungsgases hoher Reinheit;
- Aufteilen des Verdünnungsgases hoher Reinheit in erste und zweite Teile;
- Steuern des Durchflusses von zumindest einem Standardgas hoher Konzentration;
- Mischen des ersten Teils des Verdünnungsgases hoher Reinheit und des Standardgases hoher Konzentration zur Erzeugung eines Gasgemisches mittlerer Konzentration;
- Aufteilen des Gasgemisches mittlerer Konzentration in einen ersten Strom und einen zweiten Strom;
- Verdünnen des Gasgemisches des ersten Stroms mit dem zweiten Teil des Verdünnungsgases hoher Reinheit zur Erzeugung eines Gasgemisches niedriger Konzentration;
- Steuern des Drucks des zweiten Stroms des Gasgemisches mittlerer Konzentration und Steuern des Drucks des Gasgemisches niedriger Konzentration.

7. Verfahren nach Anspruch 6, zusätzlich umfassend den Schritt des Drosselns des Stroms des ersten Teils des Verdünnungsgases hoher Reinheit, das mit dem Standardgas hoher Konzentration gemischt werden soll.

8. Verfahren nach Anspruch 6 oder 7, zusätzlich umfassend den Schritt des Drosselns des ersten Stroms des Gasgemisches mittlerer Konzentration.

9. Verfahren nach einem der Ansprüche 6 bis 8, zusätzlich umfassend den Schritt des Drosselns des Stroms des zweiten Teils des Verdünnungsgases hoher Reinheit.

10. Verfahren nach einem der Ansprüche 7 bis 9, bei dem jeder Schritt zum Drosseln des Stroms durch eine Drosseleinrichtung ausgeführt wird, die aus einer kalibrierten Öffnung oder einem Nadelventil ausgewählt ist.

11. Verfahren nach einem der Ansprüche 6 bis 10, zusätzlich umfassend den Schritt des Auslassens des zweiten Stroms des Gasgemisches mittlerer Konzentration.

12. Verfahren zur Erzeugung von Gasgemischen niedriger Konzentration unter Verwendung der Vorrichtung nach einem der Ansprüche 3 bis 5, umfassend die Schritte:
- Steuern des Drucks eines Rohgases, daraufhin
- Reinigen des Rohgases zur Erzeugung eines Verdünnungsgases hoher Reinheit;
- Aufteilen des Verdünnungsgases hoher Reinheit in n Stromteile;
- Steuern des Durchflusses von zumindest einem Standardgas hoher Konzentration;
- Mischen eines ersten Stromteils des Verdünnungsgases hoher Reinheit mit dem Standardgas hoher Konzentration zur Erzeugung eines ersten Gasgemisches mittlerer Konzentration;
- Aufteilen des ersten Gasgemisches mittlerer Konzentration in einen ersten Strom und einen zweiten Strom;
- Mischen des ersten Stroms des ersten Gasgemisches mittlerer Konzentration mit einem zweiten Stromteil des Verdünnungsgases hoher Reinheit zur Erzeugung eines zweiten Gasgemisches mittlerer Konzentration;
- Aufteilen des zweiten Gasgemisches mittlerer Konzentration in einen ersten Strom und einen zweiten Strom;
- m-maliges Wiederholen der zwei vorstehend genannten Misch- und Aufteilungsschritte zur Erzeugung eines ersten und eines zweiten Stroms eines m-ten Gasgemisches mittlerer Konzentration, wobei m von zwei bis n-1 variiert;
- Mischen des ersten Stroms des (n-1)-ten Stroms des Gasgemisches mittlerer Konzentration mit dem (n)-ten Stromteil des Verdünnungsgases hoher Reinheit zur Erzeugung eines Gasgemisches niedriger Konzentration;
- Steuern des Drucks des Gasgemisches niedriger Konzentration;
- Steuern des Drucks des zweiten Stroms von jedem der ersten bis (n-1)-ten Gasgemische mittlerer Konzentration, wobei der Druck des zweiten Stroms des m-ten Gasgemisches mittlerer Konzentration (1<m<n-1) kleiner ist als derjenige des (m-1)-ten Gasgemisches mittlerer Konzentration.

13. Vorrichtung nach Anspruch 12, zusätzlich umfassend den Schritt des Drosselns des Stroms eines jeden der n Stromteile des Verdünnungsgases hoher Reinheit.

14. Verfahren nach Anspruch 12 oder 13, zusätzlich umfassend den Schritt des Drosselns der Strömung des ersten Stroms von jedem der Gasgemische mittlerer Konzentration.

15. Verfahren nach einem der Ansprüche 12 bis 14, außerdem umfassend den Schritt des Auslassens des zweiten Stroms eines jeden der Gasgemische mittlerer Konzentration.

16. Verfahren nach Anspruch 13 oder 14, wobei jeder Schritt zum Drosseln der Strömung durch eine Drosseleinrichtung ausgeführt wird, die kalibrierte Öffnungen oder Nadelventile umfaßt.

17. Verfahren nach einem der Ansprüche 6 bis 16, zusätzlich umfassend die Schritte des Steuerns des Durchflusses einer Mehrzahl von Standardgasen hoher Konzentration und Mischen der Standardgase hoher Konzentration zur Erzeugung eines Standardgasgemisches hoher Konzentration, wobei diese jeweiligen Volumenanteile gesteuert werden, wobei das Gemisch zusätzlich mit dem ersten Teil des Verdünnungsgases hoher Reinheit gemischt wird.

## Revendications

1. Dispositif de production de mélanges gazeux à basse concentration, comprenant:
- un moyen de régler la pression (2) d'un gaz brut;
- un moyen de purifier le gaz brut (3), produisant ainsi un gaz diluant à grande pureté, ledit moyen (3) étant placé en aval du moyen (2);
- un moyen de diviser (4) ledit gaz diluant à grande pureté en une première et une seconde portion, ladite seconde portion s'écoulant à travers une premier moyen restricteur (R1);
- un second moyen restricteur (R3) à travers lequel ladite première portion s'écoule;
- un moyen de régler le taux de débit (7) d'un gaz type à grande concentration;
- un moyen d'effectuer le mélange (50) de la première portion de gaz diluant à grande pureté avec le gaz type à grande concentration, produisant ainsi un mélange gazeux à concentration moyenne;
- un moyen permettant de diviser (51) le mélange gazeux à concentration moyenne en un premier débit et un second débit, ledit premier débit s'écoulant à travers un troisième moyen restricteur (R2);
- un moyen permettant de diluer (52) le mélange gazeux à concentration moyenne du premier débit avec ladite seconde portion de gaz diluant à grande pureté, produisant ainsi un mélange gazeux à basse concentration;
- un moyen permettant de régler la pression (14, 15) de ladite seconde portion de débit du mélange gazeux, réglant ainsi la pression de ladite première portion;
- un moyen permettant de régler la pression (12, 13) dudit mélange gazeux à basse concentration.

2. Dispositif selon la revendication 1, caractérisé en ce que ledit moyen restricteur de débit (R1, R3) est soit un orifice calibré ou une valve à aiguille.

3. Dispositif de production de mélanges gazeux à basse concentration, comprenant:
- un moyen permettant de régler la pression (2) d'un gaz brut;
- un moyen permettant de purifier le gaz brut (3) produisant ainsi un gaz diluant à grande pureté, ledit moyen (3) étant en aval du moyen (2);
- un moyen permettant de diviser (4) ledit gaz diluant à grande pureté en n portions de débit, chaque portion s'écoulant à travers un premier moyen restricteur (R1, R3, R5, R7);
- un moyen permettant de régler le taux de débit (7) d'un gaz type à grande concentration;
- un moyen d'effectuer le mélange (50) d'une première portion de débit du gaz diluant à grande pureté avec le gaz type à grande concentration, produisant ainsi un premier mélange gazeux à concentration moyenne;
- un moyen permettant de diviser (51) le premier mélange gazeux à concentration moyenne en un premier débit et un second débit, ledit premier débit s'écoulant à travers un second moyen restricteur (R2);
- un moyen permettant de mélanger ledit premier débit du premier mélange gazeux à concentration moyenne avec une seconde portion de débit du gaz diluant à grande pureté, produisant ainsi un second mélange gazeux à concentration moyenne;
- un moyen permettant de diviser ledit second mélange gazeux à concentration moyenne en un premier débit et un second débit;
- un moyen permettant de mélanger et un moyen permettant de diviser pour produire un premier débit et un second débit d'un (n-1)ième mélange gazeux à concentration moyenne;
- un moyen permettant de mélanger (52) le premier débit du (n-1)ième débit du gaz à concentration moyenne avec la nième portion de débit du gaz diluant à grande pureté, produisant ainsi un mélange gazeux à basse concentration;
- un moyen de régler la pression (12, 13) dudit mélange gazeux à basse concentration;
- un moyen permettant de régler la pression (14, 15, 23, 24, 33, 34) du second débit de chacun des premiers au (n-1)ième mélange gazeux à concentration moyenne.

4. Dispositif selon la revendication 3, où le premier et le second moyen restricteur de débit (R1, R2, R3, R5, R7) sont soit des orifices calibrés ou des valves à aiguille.

5. Dispositif selon la revendication 3 ou 4, comprenant en outre un moyen pour expulser le second débit de chacun des mélanges gazeux à concentration moyenne.

6. Un procédé de production de mélanges gazeux à basse concentration utilisant l'appareil selon l'une des revendications 1 ou 2 comprenant les étapes suivantes:
- régler la pression d'un gaz brut, ensuite
- purifier le gaz brut, produisant ainsi un gaz diluant à grande pureté;
- diviser ledit gaz diluant à grande pureté en une première et un seconde portions;
- régler le taux de débit d'au moins un gaz type à haute concentration;
- mélanger ladite première portion dudit gaz diluant à grande pureté avec le gaz type à haute concentration, produisant ainsi un mélange gazeux à concentration moyenne;
- diviser le mélange gazeux à concentration moyenne en un premier débit et un second débit;
- diluer le mélange gazeux du premier débit avec ladite seconde portion du gaz diluant à grande pureté, produisant ainsi un mélange gazeux à basse concentration;
- régler la pression dudit second débit de mélange gazeux moyen; et régler la pression dudit mélange gazeux à basse concentration.

7. Le procédé selon la revendication 6, comprenant en outre l'étape de restreindre le débit de la première portion de gaz diluant à grande pureté à mélanger avec le gaz type à haute concentration.

8. Le procédé selon la revendication 6 ou 7, comprenant en outre l'étape de restreindre ledit premier débit de mélange gazeux à concentration moyenne.

9. Le procédé selon l'une des revendications 6 à 8, comprenant en outre l'étape de restreindre le débit de ladite seconde portion du gaz diluant à grande pureté.

10. Le procédé selon l'une des revendications 7 à 9 où chaque étape de restriction du débit s'effectue par un moyen restricteur choisi parmi un orifice calibré ou une valve à aiguille.

11. Le procédé selon l'une des revendications 6 à 10, comprenant en outre l'étape d'expulser ledit second débit de mélange gazeux à concentration moyenne.

12. Un procédé de production de mélanges gazeux à basse concentration utilisant l'appareil selon l'une des revendications 3 à 5, comprenant les étapes suivantes:
- régler la pression d'un gaz brut, et ensuite
- purifier le gaz brut, produisant ainsi un gaz diluant à grande pureté;
- diviser le gaz diluant à grande pureté en n portions de débit;
- régler le taux de débit d'au moins un gaz type à grande concentration;
- mélanger une première portion de débit du gaz diluant à grande pureté avec le gaz type à haute concentration, produisant un premier mélange gazeux à concentration moyenne;
- diviser le premier mélange gazeux à concentration moyenne en un premier débit et un second débit;
- mélanger ledit premier débit dudit premier mélange gazeux à concentration moyenne avec une seconde portion de débit du gaz diluant à grande pureté, produisant ainsi un second mélange gazeux à concentration moyenne;
- diviser ledit second mélange gazeux à concentration moyenne en un premier débit et un second débit;
- répéter (m) fois les deux étapes ci-dessus de mélange et de division pour produire un premier et un second débit d'un (m)ième mélange gazeux à concentration moyenne, où m varie entre 2 à n-1;
- mélanger le premier débit du (n-1)ième débit du gaz à concentration moyenne avec la nième portion de débit du gaz diluant à grande pureté, produisant ainsi un mélange gazeux à basse concentration;
- régler la pression dudit mélange gazeux à basse concentration;
- régler la pression du second débit de chacun des premier au (n-1)ième mélanges gazeux à concentration moyenne, la pression dudit second débit du (m)ième mélange gazeux à concentration moyenne (1<m(n-1) étant plus petite que celle du (m-1)ième mélange gazeux à concentration moyenne.

13. Le procédé selon la revendication 12, comprenant en outre l'étape de restreindre le débit de chacune des n portions de débit du gaz diluant à grande pureté.

14. Le procédé selon la revendication 12 ou 13, comprenant en outre l'étape de restreindre le débit dudit premier débit de chacun desdits mélanges gazeux à concentration moyenne.

15. Le procédé selon l'une des revendications 12 ou 14, comprenant en outre l'étape d'expulser le second débit de chacun des mélanges gazeux à concentration moyenne.

16. Le procédé selon les revendications 13 ou 14, dans lequel chaque étape de restriction de débit s'effectue par un moyen restricteur choisi parmi des orifices calibrés ou des valves à aiguille.

17. Le procédé selon l'une quelconque des revendications 6 à 16, comprenant en outre les étapes de régler le taux de débit de plusieurs gaz type à grande concentration et de mélanger lesdits gaz types de grande concentration pour produire un mélange gazeux type à grande concentration dans des proportions respectives en volume réglées, ledit mélange étant en outre mélangé avec ladite première portion dudit gaz diluant à grande pureté.
